# EUROPEAN PATENT SPECIFICATION

(11) **EP 1 976 960 B1**
(45) Date of publication and mention of the grant of the patent: **15.07.2009**
(21) Application number: 07700488.5
(22) Date of filing: 16.01.2007
(51) Int. Cl.: C10G 3/00, C10G 21/16, C11B 7/00, C07C 67/02, C07C 69/24

(54) **PROCESS FOR THE PREPARATION OF BIODIESEL**
VERFAHREN ZUR HERSTELLUNG VON BIODIESEL
PROCEDE DE PREPARATION DE BIODIESEL

(30) Priority: 19.01.2006 IT MI20060082
(43) Date of publication of application: 08.10.2008
(73) Proprietor: MERLONI PROGETTI S.p.A., 20151 Milano (IT)
(72) Inventor: GERMANI, Maurizio, I-20158 Milano (IT)
(74) Representative: Dini, Roberto
(86) International application number: PCT/IB2007/000104
(87) International publication number: WO 2007/083213

(56) References cited:
- EP-A- 0 194 165
- WO-A-01/12581
- WO-A-20/04029016
- DE-A1- 3 015 277
- US-A- 4 164 506

## Description

The invention relates to a process for the preparation of biodiesel. In particular, the present invention relates to a process for the preparation of biodiesel which comprises the separation of the triglyceride component from the free fatty acid one, both of which are contained in at least one biolipid, the esterification of said acid component, and the joint trans-esterification of the resulting esterified acid component with said triglyceride component.

The production of methylic or ethylic esters from triglycerides and fatty acids (found in vegetable oils and animal fats) has become very important for the purpose of producing alternative fuels (biodiesel) to be used as such, alone or mixed in different ratios with mineral oil-based fuels.

The term "biodiesel" refers to a transparent or slightly amber-coloured liquid fuel wholly obtained from vegetable oils and/or animal fats, and having a viscosity similar to that of normal automotive diesel fuel.

Since the cost of raw material (biolipid) for this type of production is one of the most important factors, the industry is evidently induced to use low-value raw materials such as unrefined vegetable oils, fats recovered from slaughtered animals, virgin or recycled oleins and vegetable oils, and waste fats, all of which are substances having organic acidity due to the presence of free fatty acids, generally referred to in the industry with the acronym "FFA" (Free Fatty Acids).

Traditional trans-esterification processes cannot use oils or fats with FFA acidity exceeding 0.5% in weight (expressed as oleic acid), because said free acidity, in addition to consuming the basic catalyst (generally potassium hydrate or sodium methylate), also produces, by reacting with the basic catalyst, soaps which interfere with the production of methylesters, thus creating complications and difficulties due to the necessary separation of said soaps from the methylesters, which undo the potential benefits deriving from the use of said less expensive raw materials.

Some recently proposed processes, which include a preliminary FFA esterification with methanol, followed by trans-esterification of the unreacted triglyceride fraction, in turn suffer from the necessity of esterifying small acid fractions in the presence of large quantities of triglycerides, and therefore require the use of large reactors involving considerable installation and operating costs.

Surprisingly, it has been found lately that the above-described problems can be advantageously solved by implementing a process for the preparation of biodiesel which comprises the following sequential and continuous steps:
a) separation of the triglyceride component from the free fatty acid ones, both of which are contained in at least one biolipid, by extracting said acid component with methanol;
b) esterification of said acid component with methanol;
c) reunion of the resulting esterified acid component with said triglyceride component, and joint trans-esterification thereof in the presence of excess methanol and of at least one basic catalyst.

In the present description, the term "biolipid" refers to a virgin and/or waste (e.g. used oil, recycled oil, slaughtering waste) vegetable oil or animal fat from any source. In particular, the biolipid is selected from the group including apricot oil, cashew, peanut, avocado, hemp, colza, cotton, rice bran, sunflower, linen, linseed, maize, almond, hazelnut, walnut, coconut, olive, palm, poppy, castor bean, mustard, sesame, soy, soy bean, pumpkin, or an animal fat, e.g. tallow, all of which may be virgin and/or waste feedstock.

According to a preferred embodiment, the extraction is carried out through a pulsating filling column at a temperature of about 40-60°C, with a methanol/biolipid weight ratio ranging between about 1.2 and 2.0; in particular, the column is filled in a structured manner.

It is also preferable to carry out the esterification of the acid component, during which the previously extracted free fatty acids are turned into methylesters, in the presence of at least one heterogeneous acid catalyst, e.g. selected from the group including a cationic resin in acid or mineral form, such as, for example, hydrocalcites and, in particular, a cationic resin in acid form.

According to another preferred embodiment example, the esterification of the acid component is carried out in a vertical column-type esterification reactor at a temperature of about 60°C-110°C; it is also preferable to extract, continuously and in the vapour phase, a percentage of 50-80% in weight of the methanol used for carrying out step b) from the head of the esterification reactor, which percentage is then anhydrified, e.g. through distillation or adsorption on a dehydrating agent, for example and preferably molecular sieves or Drierite.

The basic catalyst is preferably selected from the group including sodium methylate, potassium hydrate, barium hydrate; preferably sodium methylate in a quantity of about 0.3-1 % in weight relative to the weight of the separated triglyceride component.

According to a further preferred embodiment example, step c) is carried out in a tubular trans-esterification reactor at a temperature of about 70°C-100°C for permanence times of about 30-90 minutes, wherein excess methanol is equal to 300-500% by weight relative to the stoichiometric quantity of the separated triglyceride component.

In particular, the basic catalyst is neutralized with carbon dioxide, downstream of the trans-esterification reactor, at a partial pressure of at least 1 bar.

It has been observed that the process according to the present invention allows to separate the two components, i.e. the acid one, substantially consisting of free fatty acids (FFA), and the triglyceride one, in an economic and selective manner, so that they can be subsequently subjected to esterification and trans-esterification, respectively.

The separation of the two components is carried out by implementing an extraction process using the esterification and trans-esterification reagent itself, i.e. methanol, as a solvent.

The process according to the invention also allows to use any biolipid, without any limitation related to the percentage of FFA acidity (expressed as oleic acid); additionally, the implementation thereof allows to avoid soap formation, without any interference with the production of methylesters resulting from trans-esterification, thereby reducing the running costs of the production plant significantly.

According to a preferred embodiment example of the process of the invention, said at least one virgin or waste biolipid (raw material), intended for biodiesel production, is subjected to a methanol-based extraction process, preferably carried out in an extraction column wherein both raw material and methanol flows are fed continuously according to a weight ratio between the methanol flow rate and the raw material flow rate comprised between 1.2 and 2.0, preferably 1.5.

Said values derive from the free fatty acid distribution coefficient in the methanol and triglyceride phases, which has an approximately unitary value (expressed as a ratio between the weight concentrations of free fatty acids in the two phases).

The extraction device, which may be suitably selected by those skilled in the art, is preferably a continuous extraction column of the holed-plate or filling type. In particular, it has been observed that higher efficiency is obtained with a pulsating filling column.

In order to achieve good extraction results, the methanol must be sufficiently anhydrous (water content preferably lower than 0.15% by weight).

The extraction column operates at a sufficiently high temperature to ensure good fluidity of the biolipid to be treated (typically 40°C-60°C) and at atmospheric pressure. It is also possible to operate at higher temperatures (60°C-100°C) by performing extraction under pressure, but no significant variation of the distribution coefficient would be obtained.

The methanol phase, which has extracted the acid component (FFA), is not actually totally inert towards the triglyceride component. In fact, it has been observed that about 20% by weight of triglycerides (relative to the mass of the extracted fatty acids) remains in the methanol phase.

However, this is not a problem for the production of methylesters, in that said triglyceride fraction, which is co-extracted in the methanol phase, goes unchanged through the esterification of the acid fraction and is also transformed into methylesters in the subsequent esterification phase.

The organic (triglyceride) phase flowing out of the bottom of the extraction column has a residual acidity content which can be adjusted to a value being similar to that of a refined biolipid (0.1-0.2% by weight, expressed as oleic acid), and is therefore suitable for being fed to trans-esterification. This organic phase also contains a small weight percentage of methanol (1-2%) which does not affect trans-esterification in any way, since methanol is also the trans-esterification reagent.

The esterification reaction of the fatty acids extracted in the methanol phase is preferably carried out in a static column-type reactor in the presence of an acid catalyst, preferably an ionic-exchange cationic resin in acid form, at a temperature of 60°C-110°C and at a corresponding pressure (approximately 0 to 5 bar) due to the vapour tension of methanol and to the load losses of the system.

The time of permanence in the reactor (of the mass of methanol plus the mass of the extracted free fatty acids) is between 30 minutes and 2 hours, preferably 45 minutes, at 100°C.

During esterification, water is produced due to the quantity of free acidity being present. This water, which would be found in the methanol exiting the esterification reactor, must be kept at a level lower than 0.15% by weight relative to methanol (preferably less than 500 ppm by weight) in order to obtain good trans-esterification results.

For this purpose, a percentage (50-80% by weight) of methanol in the gaseous phase is taken from the reactor head and is then sent to a distillation column from the bottom of which water flows out, whereas anhydrous methanol is taken from the column head and is then reintroduced, together with fresh methanol, into the trans-esterification unit, wherein the triglyceride fraction (previously separated by extraction) and the esterified methanol phase are made to flow together.

The trans-esterification reaction is preferably carried out in a tubular reactor such as, for example, the one described in Italian Patent IT 1 255 466, for a permanence time between 30 and 90 minutes, at a temperature between 60°C and 120°C and at a pressure corresponding to the vapour tension of methanol at the reaction temperature. Preferred conditions are a temperature of 80°C and a pressure of 3 bar. The catalyst used is a strong base (e.g. sodium methylate, potassium hydrate, barium hydrate; preferably sodium methylate) at the rate of 0.3-1% relative to the triglyceride mass. Methanol is present with a 20-50% excess weight relative to the weight of the triglyceride component.

The reaction mixture is then neutralized with carbon dioxide under moderate pressure (2-5 bar) in order to provide the corresponding carbonates of the base used as a catalyst.

After neutralization, excess methanol is recovered and glycerin is then separated from methylester, which steps take place according to common techniques known to those skilled in the art.

The following examples illustrate the invention without any limitation thereto.

### Example 1

10 kg of raw palm oil having a free acidity of 5% were extracted continuously with 14.1 kg of methanol in a glass column being 3m long and having a diameter of 50mm, filled with Rashig rings being about 10mm thick.

A methanol phase containing 97% of the initial acidity and an organic phase with 0.15% of residual free acidity (expressed as oleic acid) were obtained.

The methanol phase was fed into a cylindrical reactor having a diameter of 50mm, regulated at 70°C by a thermostat and filled with about 1 litre of Amberlyst^{®} 15 dry strong cationic resin produced by Rohm &Haas.

During the operation, approximately 70% of methanol was taken from the reactor head and subsequently anhydrified.

The esterified methanol phase was added to the triglyceride fraction and was then subjected to trans-esterification in the presence of sodium methylate as a catalyst, by using a tubular reactor having a diameter of 25mm and being 3m long, at a temperature of 80°C.

The progress of this test is shown in the following table

| PHASE | Triglicerides (kg) | Fatty acids (kg) | Methylesters (kg) | Water (kg) | Methanol (kg) | Glycerin/other/catalyst (kg) |
|---|---|---|---|---|---|---|
| Raw material | 9.500 | 0.500 | 0 | negligible | 0 | 0 |
| Extracted methanol phase | 0.097 | 0.485 | 0 | negligible | 14 kg | 0 |
| Extracted organic phase | 9.403 | 0.015 | 0 | negligible | 0.1 kg | 0 |
| Esterified methanol phase | 0.097 | 0 | 0.509 | 0.00928 | 4.144 kg | 0 |
| Methanol to anhydrification | | | | 0.0217 | 9.8 kg | 0 |
| Feed to trans-esterification | 9.500 | 0.015 | 0.509 | 0.0217 | 14.1 | 0/0/0.0095 |
| Reaction crude | 0 | 0 | 9.901 | 0.0217 | 13.068 | 0.010/0.150 /0.0095 |

At the end of the process, methylester (biodiesel) was obtained according to the EN 14214 Specification as far as the contents of methylester, monoglycerides, diglycerides and triglycerides are concerned.

### Example 2

10 kg of raw colza oil having a free acidity of 3% were extracted continuously with 14.1 kg of methanol in a glass column being 3m long and having a diameter of 50mm, filled with Rashig rings being about 10mm thick.

A methanol phase containing 97% of the initial acidity and an organic phase with 0.14% of residual free acidity (expressed as oleic acid) were obtained.

The methanol phase was fed into a cylindrical reactor having a diameter of 50mm, regulated at 70°C by a thermostat and filled with about 1 litre of Amberlyst^{®} 15 dry strong cationic resin produced by Rohm &Haas.

During the operation, approximately 70% of methanol was taken from the reactor head and subsequently anhydrified.

The esterified methanol phase was added to the triglyceride fraction and was then subjected to trans-esterification in the presence of sodium methylate as a catalyst, by using a tubular reactor having a diameter of 25mm and being 3m long, at a temperature of 80°C.

The progress of this test is shown in the following table

| PHASE | Triglicerides (kg) | Fatty acids (kg) | Methylesters (kg) | Water (kg) | Methanol (kg) | Glycerin/other/catalyst (kg) |
|---|---|---|---|---|---|---|
| Raw material | 9.700 | 0.300 | 0 | negligible | 0 | 0 |
| Extracted methanol phase | 0.056 | 0.290 | 0 | negligible | 14 kg | 0 |
| Extracted organic phase | 9.644 | 0.010 | 0 | negligible | 0.1 kg | 0 |
| Esterified methanol phase | 0.056 | 0 | 0.304 | 0.00555 | 4.144 kg | 0 |
| Methanol to anhydrification | | | | 0.0129 | 9.8 kg | 0 |
| Feed to trans-esterification | 9.644 | 0.010 | 0.304 | 0.0129 | 14.0 | 0/0/0.0095 |
| Reaction crude | 0 | 0 | 9.901 | 0.0217 | 13.068 | 0.010/0.150 /0.0095 |

At the end of the process, methylester (biodiesel) was obtained according to the EN 14214 Specification as far as the contents of methylester, monoglycerides, diglycerides and triglycerides are concerned.

## Claims

1. Process for the preparation of biodiesel, which comprises the following sequential and continuous steps:
a) separation of the triglyceride component from the free fatty acid one, both of which are contained in at least one biolipid, by extracting said acid component with methanol;
b) esterification of said acid component with methanol;
c) reunion of the resulting esterified acid component with said triglyceride component, and joint trans-esterification thereof in the presence of excess methanol and of at least one basic catalyst.

2. Process according to claim 1, wherein the biolipid is vegetable oil or animal fata virgin and/or waste vegetable oil or an animal fat.

3. Process according to claim 1 o 2, wherein the biolipid is selected from the group including apricot oil, cashew, peanut, avocado, hemp, colza, cotton, rice bran, sunflower, linen, linseed, maize, almond, hazelnut, walnut, coconut, olive, palm, poppy, castor bean, mustard, sesame, soy, soy bean, pumpkin, and tallow, all of which may be virgin and/or waste.

4. Process according to any of the preceding claims, wherein the extraction is carried out by using a pulsating filling column at a temperature of 40°C-60°C, with a methanol/biolipid weight ratio ranging between 1.2 and 2.0.

5. Process according to the previous claim, wherein the column is filled in a structured manner.

6. Process according to any of the previous claims, wherein step b) is carried out in the presence of at least one heterogeneous acid catalyst.

7. Process according to the previous claim, wherein the heterogeneous catalyst is a cationic resin in acid form.

8. Process according to any of the previous claims, wherein step b) is carried out in a vertical column-type esterification reactor at a temperature of 60°C-110°C.

9. Process according to the previous claim, wherein a percentage of 50-80% by weight of the methanol used for carrying out step b) is extracted continuously from the esterification reactor head, in the vapour phase, to be subsequently anhydrified on a dehydrating agent through distillation or adsorption.

10. Process according to any of the previous claims, wherein the basic catalyst is selected from the group including sodium methylate, potassium hydrate, barium hydrate, at the rate of 0.3-1% by weight relative to the weight of the separated triglyceride component.

11. Process according to any of the previous claims, wherein step c) is carried out in a tubular trans-esterification reactor at a temperature of 70°C-100°C for permanence times of 30-90 minutes, and wherein excess methanol is 300-500% by weight relative to the stoichiometric quantity of the separated triglyceride component.

12. Process according to the previous claim, wherein the basic catalyst is neutralized with carbon dioxide, downstream of the tran-sesterification reactor, at a partial pressure of at least 1 bar.

## Patentansprüche

1. Verfahren zur Herstellung von Biodiesel, das die folgenden sequentiellen und kontinuierlichen Schritte umfasst:
a) Trennung der Triglyceridkomponente von der freien Fettsäurekomponente, die beide wenigstens in einem Biolipid enthalten sind, durch Extrahieren besagter saurer Komponente mit Methanol;
b) Veresterung der besagten sauren Komponente mit Methanol;
c) Wiedervereinigung der resultierenden veresterten sauren Komponente mit besagter Triglyceridkomponente und gemeinsame Transveresterung von ihnen in Gegenwart von einem Überschuss an Methanol und wenigstens von einem basischen Katalysator.

2. Verfahren gemäß Anspruch 1, wobei das Biolipid Pflanzenöl oder tierisches Fett, natives und/oder Pflanzenölabfall oder ein tierisches Fett ist.

3. Verfahren gemäß der Ansprüche 1 oder 2, wobei das Biolipid ausgewählt ist aus der Gruppe umfassend Aprikosen-, Cashewnuss-, Erdnuss-, Avocado-, Hanf-, Raps-, Baumwoll-, Reiskleie-, Sonnenblumen-, Leinen-, Leinsamen-, Mais-, Mandel-, Haselnuss-, Walnuss-, Kokosnuss-, Oliven-, Palmen-, Mohn-, Rizinussamen-, Senf-, Sesam-, Soja-, Sojabohnen-, Kürbis-, und Talgöl, von denen alle nativ und/oder gebraucht sein können.

4. Verfahren gemäß einem der vorherigen Ansprüche, wobei die Extraktion durchgeführt wird durch die Benutzung einer Pulsierfüllkolonne bei einer Temperatur von 40°C-60°C mit einem Methanol/Biolipid-Gewichts-Verhältnis im Bereich zwischen 1,2 und 2,0.

5. Verfahren gemäß dem voranstehenden Anspruch, wobei die Kolonne in einer strukturierten Weise gefüllt wird.

6. Verfahren gemäß einem der vorangehenden Ansprüche, wobei Schritt b) in Anwesenheit von wenigstens einem heterogenen sauren Katalysator durchgeführt wird.

7. Verfahren gemäß einem der vorangehenden Ansprüche, wobei der heterogene Katalysator ein kationisches Harz in saurer Form ist.

8. Verfahren gemäß einem der vorangehenden Ansprüche, wobei Schritt b) in einem senkrechten Kolonnen-Veresterungs-Reaktor bei einer Temperatur von 60°C-110°C durchgeführt wird.

9. Verfahren gemäß dem voranstehenden Anspruch, wobei ein Anteil von 50-80 Gew.-% von dem für die Durchführung von Schritt b) benutzten Methanol kontinuierlich von dem Veresterungsreaktorkopf in der Gasphase extrahiert wird, um danach über eine dehydrierende Substanz durch Destillation oder Adsorption entwässert zu werden.

10. Verfahren gemäß einem der vorangehenden Ansprüche, wobei der basische Katalysator ausgewählt ist aus der Gruppe umfassend Natriummetyhlat, Kaliumhydrat, Bariumhydrat, mit dem Anteil von 0,3-1 Gew.-% relativ zu dem Gewicht der abgetrennten Triglyceridkomponente.

11. Verfahren gemäß einem der vorangehenden Ansprüche, wobei Schritt c) in einem röhrenförmigen Trans-Veresterungs-Reaktor bei einer Temperatur von 70°C-100°C für Anwesenheitszeiten von 30-90 Minuten durchgeführt wird, und wobei der Überschuss Methanol 300-500 Gew.-% relativ zu der stöchiometrischen Menge der abgetrennten Triglyceridkomponente ist.

12. Verfahren gemäß dem voranstehenden Anspruch, wobei der basische Katalysator nach dem Trans-Veresterungs-Reaktor neutralisiert wird durch Kohlendioxid, mit einem Partialdruck von wenigstens 1 bar.

## Revendications

1. Procédé pour la préparation de biodiesel, qui comprend les étapes séquentielles et continues suivantes :
a) séparation du composant triglycéride du composant acide gras libre, les deux étant contenus dans au moins un biolipide, par l'extraction dudit composant acide avec du méthanol ;
b) estérification dudit composant acide avec du méthanol ;
c) réunion du composant acide estérifié résultant avec ledit composant triglycéride, et transestérification conjointe de celui-ci en présence d'un excès de méthanol et d'au moins un catalyseur basique.

2. Procédé selon la revendication 1, dans lequel le biolipide est une huile végétale ou une huile vierge de graisse animale et/ou une huile végétale usagée ou une graisse animale.

3. Procédé selon la revendication 1 ou 2, dans lequel le biolipide est choisi parmi le groupe comprenant l'huile d'abricot, de noix de cajou, d'arachide, d'avocat, de chanvre, de colza, de coton, de riz, de tournesol, de lin, de graines de lin, de maïs, d'amande, de noisette, de noix, de noix de coco, d'olive, de palme, de pavot, de ricin, de moutarde, de sésame, de soja, de fèves de soja, de citrouille, et de suif, toutes pouvant être vierges et/ou usagées.

4. Procédé selon l'une quelconque des revendications précédentes, dans lequel l'extraction est réalisée en utilisant une colonne à garnissage à impulsions à une température de 40°C à 60°C, avec un rapport en poids de méthanol/biolipide compris entre 1,2 et 2,0.

5. Procédé selon la revendication précédente, dans lequel la colonne est remplie d'une manière structurée.

6. Procédé selon l'une quelconque des revendications précédentes, dans lequel l'étape b) est réalisée en présence d'au moins un catalyseur acide hétérogène.

7. Procédé selon la revendication précédente, dans lequel le catalyseur hétérogène est une résine cationique sous forme acide.

8. Procédé selon l'une quelconque des revendications précédentes, dans lequel l'étape b) est réalisée dans un réacteur d'estérification de type colonne verticale à une température de 60°C à 110 °C.

9. Procédé selon la revendication précédente, dans lequel un pourcentage de 50 à 80 % en poids de méthanol utilisé pour réaliser l'étape b) est extrait de manière continue par la tête du réacteur d'estérification, dans la phase vapeur, pour être ensuite rendu anhydre sur un agent déshydratant par distillation ou adsorption.

10. Procédé selon l'une quelconque des revendications précédentes, dans lequel le catalyseur basique est choisi parmi le groupe comprenant le méthylate de sodium, l'hydrate de potassium, l'hydrate de baryum, au rapport de 0,3 % à 1 % en poids par rapport au poids du composant triglycéride séparé.

11. Procédé selon l'une quelconque des revendications précédentes, dans lequel l'étape c) est réalisée dans un réacteur de transestérification tubulaire à une température de 70°C à 100°C pendant un temps de séjour de 30 à 90 minutes, et où le méthanol en excès représente 300 % à 500 % en poids par rapport à la quantité stoechiométrique du composant triglycéride séparé.

12. Procédé selon la revendication précédente, dans lequel le catalyseur basique est neutralisé avec du dioxyde de carbone, en aval du réacteur de transestérification, à une pression partielle d'au moins 1 bar.
